(19) Europäisches Patentamt
European Patent Office
Office européen des brevets

(11) Veröffentlichungsnummer: **0 474 045 B1**

(12) **EUROPÄISCHE PATENTSCHRIFT**

(45) Veröffentlichungstag der Patentschrift: **18.10.95**

(51) Int. Cl.[6]: **C07D 405/06**, C07D 303/04, A01N 43/653, A01N 43/50

(21) Anmeldenummer: **91114061.4**

(22) Anmeldetag: **22.08.91**

(54) **Azolylmethyloxirane und diese enthaltende Fungizide.**

(30) Priorität: **07.09.90 DE 4028392**

(43) Veröffentlichungstag der Anmeldung:
**11.03.92 Patentblatt 92/11**

(45) Bekanntmachung des Hinweises auf die Patenterteilung:
**18.10.95 Patentblatt 95/42**

(84) Benannte Vertragsstaaten:
**AT BE CH DE DK ES FR GB GR IT LI NL SE**

(56) Entgegenhaltungen:
**EP-A- 0 388 871**
**EP-A- 0 431 450**
**DE-A- 3 218 130**
**FR-A- 1 374 883**

**CHEMICAL ABSTRACTS, Band 88, Nr. 21, 22. Mai 1978, Columbus, Ohio, USA SANTELLI, M. et al. "Formation of beta-bromo- -epoxides by halogenation of allylic alcohols" Seite 580, Spalte 1, Zu- sammenfassung-Nr. 152 315m**

(73) Patentinhaber: **BASF Aktiengesellschaft**
**Carl-Bosch-Strasse 38**
**D-67063 Ludwigshafen (DE)**

(72) Erfinder: **Seele, Rainer, Dr.**
**Leiblstrasse 3**
**W-6701 Fussgoenheim (DE)**
Erfinder: **Rentzea, Costin, Dr.**
**Richard-Kuhn-Strasse 1-3**
**W-6900 Heidelberg (DE)**
Erfinder: **Ammermann, Eberhard, Dr.**
**Sachsenstrasse 3**
**W-6700 Ludwigshafen (DE)**
Erfinder: **Lorenz, Gisela, Dr.**
**Erlenweg 13**
**W-6730 Neustadt (DE)**

**EP 0 474 045 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 90, Nr. 19, 7. Mai 1979, Columbus, Ohio, USA SANTELLI, M. et al. "Hydrolysis of oxirylcarbinyl tolylsulfonates" Seite 545, Spalte 2, Zusammenfassung-Nr. 151 435e

CHEMICAL ABSTRACTS, Band 98, Nr. 11, 14. März 1983, Columbus, Ohio, USA INSTITUTE OF PHYSICAL AND CHEMICAL RESEARCH "erythro-Hydroxyepoxide compounds" Seite 538, Spalte 1, Zusammenfassung-Nr. 89 147x

CHEMICAL ABSTRACTS, Band 53, Nr. 13, 10. Juli 1959, Columbus, Ohio, USA HOWARD E. ZIMMERMAN et al. "Overlap control of carbanionoid reactions. I. Stereoselectivity in alkaline epoxidation" Spalte 12162, Zusammenfassung-Nr. 12 163g

CHEMICAL ABSTRACTS, Band 52, Nr. 21, 10. November 1958, Columbus, Ohio, USA HERBERT O. HOUSE AND ROLLAND S. RO "The stereochemistry of base-catalyzed epoxidation" Spalte 18294, Zusammenfassung-Nr. 18 295b

**Beschreibung**

Die vorliegende Erfindung betrifft neue Azolylmethyloxirane, Verfahren zu ihrer Herstellung und diese enthaltende Fungizide.

Es wurde gefunden, daß Azolylmethyloxirane der allgemeinen Formel I

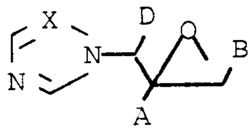

I

in welcher

A und B $C_1$-$C_8$-Alkyl, $C_3$-$C_8$-Cycloalkyl, $C_5$-$C_8$-Cycloalkenyl, Biphenyl, Naphthyl, 5-gliedriges Heteroaryl mit einem oder zwei der Heteroatome O, S und N, 6-gliedriges Heteroaryl mit bis zu drei Stickstoffatomen oder Phenyl bedeuten, wobei jeder dieser Reste ein- bis dreifach durch Halogen, Nitro, Phenoxy, Amino, $C_1$-$C_4$-Alkyl, $C_1$-$C_4$-Alkoxy oder $C_1$-$C_4$-Halogenalkyl substituiert sein kann,

D den Rest $C_1$-$C_8$-Alkyl bedeutet,

X den Rest CH oder N bedeutet,

sowie deren für Pflanzen verträgliche Säureadditionssalze und Metallkomplexe eine bessere fungizide Wirkung besitzen als bekannte Azolverbindungen.

Die Verbindungen der Formel I enthalten chirale Zentren und werden im allgemeinen in Form von Racematen bzw. als Diasteromerengemische von erythro- bzw. threo-Formen erhalten. Die erythro- bzw. threo-Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Aus einem solchen isolierten Diastereomeren kann man mit bekannten Methoden einheitliche Enantiomere erhalten. Als fungizide Mittel kann man sowohl die einheitlichen Diastereomere bzw. Enantiomere wie auch deren bei der Synthese anfallende Gemische verwenden. Alle diese Verbindungen werden von der Erfindung umfaßt.

Bevorzugt sind diejenigen Azolylmethyloxirane, in denen die Reste A und B folgende Bedeutung haben:
$C_1$- bis $C_8$-Alkyl, vorzugsweise $C_1$- bis $C_4$-Alkyl, insbesondere Methyl, Ethyl, n-Propyl, Isopropyl, n-Butyl, sec.-Butyl und tert. -Butyl; von den $C_1$- bis $C_8$-Alkylresten, die mehr als 4 C-Atome aufweisen, sind als bevorzugt n-Pentyl und Neopentyl zu nennen;
Phenyl und halogensubstituiertes Phenyl wie 2-Chlorphenyl, 3-Chlorphenyl, 4-Chlorphenyl, 2-Fluorphenyl, 3-Fluorphenyl, 4-Fluorphenyl sowie 2-Bromphenyl, 3-Bromphenyl und 4-Bromphenyl, 2,3-Dichlorphenyl, 2,4-Dichlorphenyl, 2,5-Dichlorphenyl und 2,6-Dichlorphenyl sowie 2-Chlor-4-fluorphenyl und 2-Chlor-6-fluorphenyl;
einfach durch Nitro-, Phenoxy-, Amino- und $C_1$- bis $C_4$-Alkylgruppen substituiertes Phenyl, wie 3-Nitrophenyl, 4-Nitrophenyl, 3-Phenoxyphenyl, 4-Phenoxyphenyl, 3-Aminophenyl und 4-Aminophenyl sowie 4-Ethylphenyl, 4-Isopropylphenyl und 4-tert. -Butylphenyl;
durch zwei oder drei der bereits erwähnten, aber verschiedenartigen Reste substituiertes Phenyl, wie 2-Chlor-6-methylphenyl;
einfach oder zweifach durch $C_1$- bis $C_4$-Alkoxygruppen substituiertes Phenyl, wie 2-Methoxyphenyl, 3-Methoxyphenyl, 4-Methoxyphenyl, 4-tert. -Butyloxyphenyl und 2,4-Dimethoxyphenyl sowie 3,4-Dimethoxyphenyl;
p-Biphenyl,
1-Naphthyl und 2-Naphthyl;
5-gliedriges Hetereoaryl mit einem oder zwei der Heteroatome O, S und N, insbesondere Furyl, 2-Furyl, Thienyl, 2-Thienyl, 3-Thienyl, Oxazolyl, 4-Oxazolyl, Thiazolyl, 4-Thiazolyl, Isoxazolyl, 4-Isoxazolyl, 5-Isoxazolyl und Imidazolyl, 5-Imidazolyl;
6-gliedriges Heteroaryl mit bis zu drei Stickstoffatomen wie Pyridinyl, 2-Pyridinyl, 3-Pyridinyl und 4-Pyridinyl;
$C_3$- bis $C_8$-Cycloalkyl, vorzugsweise Cyclopentyl und Cyclohexyl;
$C_5$- bis $C_8$-gycloalkenyl, vorzugsweise 3-Cyclohexenyl.

Der Rest D steht für
$C_1$- bis $C_8$-Alkyl, vorzugsweise $C_1$- bis $C_4$-Alkyl, insbesondere Methyl, Ethyl, n-Propyl, Isopropyl, n-Butyl,

sec.-Butyl und tert.-Butyl; von den $C_1$- bis $C_8$-Alkylgruppen, die mehr als 4 C-Atome aufweisen, sind als bevorzugt n-Pentyl und Neopentyl zu nennen.

Säureadditionssalze sind beispielsweise die Hydrochloride, Bromide, Sulfate, Nitrate, Phosphate, Oxalate, oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der Azolylmethyloxirane (I) mit geeigneten Säuren.

Metallkomplexe der Wirkstoffe I oder ihrer Salze können mit Kupfer, Zink, Zinn, Mangan, Eisen, Kobalt oder Nickel gebildet werden, indem man die Azolylmethyloxirane mit entsprechenden Metallsalzen umsetzt.

Die Verbindungen der Formel I können z.B. hergestellt werden, indem man

a) eine Verbindung der Formel II

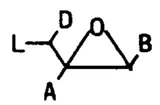

II,

in welcher A, B und D die oben angegebene Bedeutung haben und L eine nukleophile Abgangsgruppe darstellt, mit einer Verbindung der Formel III

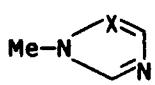

III,

in der Me ein Wasserstoffatom oder ein Metallatom bedeutet und X die angegebene Bedeutung hat, zur Umsetzung bringt, oder

b) eine Verbindung der Formel IV

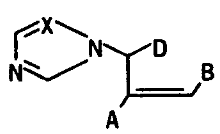

IV,

in welcher A, B, D und X die oben angegebene Bedeutung haben, in die Epoxide überführt.

Die Reaktion a) erfolgt - falls Me ein Wasserstoffatom bedeutet - gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium- oder Kaliumcarbonat oder Natrium- oder Kaliumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid oder Kaliumjodid, quaternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-krone-6 oder Dicyclohexano-18-krone-6 in Frage.

Die Umsetzung wird im allgemeinen bei Temperaturen zwischen 20 und 150 °C, drucklos oder unter Druck, kontinuierlich oder diskontinuierlich durchgeführt.

Ist Me ein Metallatom wird die Reaktion a) gegebenenfalls in Gegenwart eines Lösungs- oder Verdünnungsmittels und gegebenenfalls unter Zusatz einer starken anorganischen oder organischen Base bei Temperaturen zwischen -10 und 120 °C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethylphosphortriamid, Sulfoxide wie Dimethylsulfoxid und Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert. -butoxid.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen zwischen 0 und 100 °C, vorzugsweise bei 20 bis 80 °C. Bei Anwesenheit eines Lösungsmittels wird zweckmäßigerweise beim Siedepunkt des jeweiligen Lösungsmittels gearbeitet.

Die Ausgangsverbindungen II erhält man durch Epoxidierung der entsprechenden Olefine V:

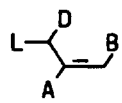

V

(vgl. G. Dittus in Houben-Weyl-Müller, Methoden der organischen Chemie, Georg Thieme Verlag, Stuttgart, 1965, Bd. VI, 3, Seite 385 ff).

Die Verbindung V stellt man her, indem man Olefine der Formel VI

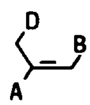

VI

nach bekannten Methoden in Allylposition halogeniert oder oxidiert.

Geeignete Halogenierungsreagenzien sind N-Chlor- und N-Bromsuccinimid in halogenierten Kohlenwasserstoffen wie Tetrachlorkohlenstoff, Trichlorethan oder Methylenchlorid bei Temperaturen zwischen 20 und 100 °C. Zur Allyloxidation verwendet man Perester wie Perbenzoesäure-tert. -butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100 °C.

Die so erhaltenen Allylhalogenide bzw. -alkohole V werden anschließend in die entsprechenden Epoxide II (L = Halogen, OH) übergeführt. Dazu oxidiert man die Olefine V mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat. Man arbeitet zwischen 10 und 100 °C und katalysiert die Reaktion gegebenenfalls z.B. mit Iod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30 °C sowie Alkylhydroperoxide, z.B. tert. -Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Die Verbindungen der allgemeinen Formel II

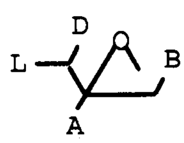

II

ausgenommen

a) 3,4-Epoxy-3-methyl-2-brompentan,

b 3,4-Epoxy-3-methyl-2-(4-methylphenylsulfonyloxy)-pentan,

c) 3,4-Epoxy-3-methyl-2-pentanol,

d) 4,5-Epoxy-4-methyl-3-heptanol und
e) 3,4-Diphenyl-3,4-epoxy-2-butanol
sind neu.

Während die so erhaltenen Epoxihalogenide II (L = Halogen) gemäß Verfahren a) sofort umgesetzt werden können, überführt man die entsprechenden Epoxialkohole II (L = OH) in reaktive Ester, die dann mit den Verbindungen III gemäß Verfahren a) umgesetzt werden.

Die Darstellung der reaktiven Ester, die mit III umgesetzt werden, erfolgt nach allgemein bekannten Methoden (Houben-Weyl-Müller, Methoden der organischen Chemie, Georg Thieme Verlag, Stuttgart, 1955, Band 9, Seiten 388, 663, 671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Verbindungen IV werden beispielsweise mit Oxidationsmitteln in ihrer Epoxide überführt.

Die Verbindungen V lassen sich entsprechend allgemein bekannten Verfahren zur Olefinsynthese (Houben-Weyl-Müller, Methoden der organischen Chemie, Georg Thieme Verlag, Stuttgart, 1972, Bd. V, 1b) herstellen.

Die folgenden Beispiele erläutern die Herstellung der Wirkstoffe.

I. Herstellung der Ausgangsstoffe

Vorschrift A

1-(4-Fluorphenyl)-2-(3-trifluormethylphenyl)-buten-2-on

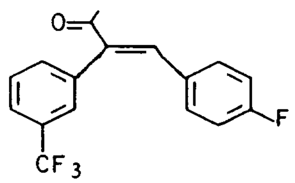

Zu einer Lösung von 50 g (0,247 mol) 3-Trifluormethylphenylaceton in 250 ml Toluol werden 27,8 g (0,224 mol) 4-Fluorbenzaldehyd und 2 ml Piperidin gegeben und unter Auskreisung des Reaktionswassers erhitzt. Anschließend wird die Reaktionslösung einmal mit 0,5 M Salzsäure und dreimal mit Wasser gewaschen, über Natriumsulfat getrocknet und im Vakuum eingedampft. Bei der anschließenden Destillation des verbleibenden Rückstandes werden bei 0,1 mbar und 102 °C Übergangstemperatur 62 g (90 %) Produkt erhalten.

Vorschrift B

cis-2-(1-Hydroxyethyl)-2-(3-trifluormethylphenyl)-3-(4-fluorphenyl)-oxiran

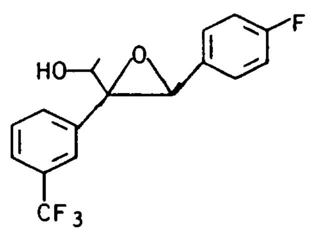

62 g (0,2 mol) 1-(4-Fluorphenyl)-2-(3-trifluormethylphenyl)-buten-2-on werden in 300 ml Methanol gelöst und 4 ml Natronlauge (konz.) zugesetzt. Die Reaktionslösung wird bei 0 °C gerührt, während 16,4 g Wasserstoffperoxid langsam zugetropft werden, wobei die Innentemperatur von 30 °C nicht überschritten wird. Nach beendeter Zugabe wird sechs Stunden bei Raumtemperatur gerührt und anschließend 2,5 g

Natriumborhydrid zugegeben, das in wenig 10 %iger Natronlauge gelöst ist. Nachdem das Reaktionsgemisch 18 Stunden bei Raumtemperatur gerührt worden war, wird der Lösung 100 ml Wasser zugesetzt und die entstandene Emulsion mit Methylenchlorid ausgeschüttelt. Die isolierte organische Phase wird daraufhin über Natriumsulfat getrocknet und eingeengt. Man erhält 60 g (92 %) Produkt als 1:1-Diastereomerengemisch.

Vorschrift C

cis-2-(1-Methylsulfonyloxyethyl)-2-(3-trifluormethylphenyl)-3-(4-fluorphenyl)-oxiran

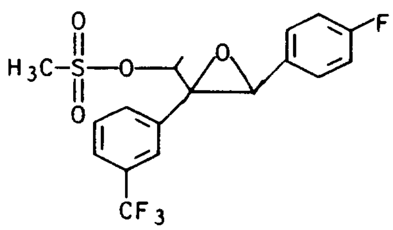

Zu einer Lösung von 40 g (0,123 mol) cis-2-(1-Hydroxyethyl)-2-(3-trifluormethylphenyl)-3-(4-fluorphenyl)-oxiran in 200 ml Methylenchlorid und 21,5 ml Triethylamin werden bei Raumtemperatur 17,6 g Methansulfonsäurechlorid gegeben. Nach 24 Stunden wird das Reaktionsgemisch mit wäßriger Natriumhydrogencarbonatlösung und Wasser gewaschen, über Natriumsulfat getrocknet und im Vakuum eingedampft. Aus dem Rückstand erhält man 45 g (91 %) Produkt als 1:1-Diastereomerengemisch.

II. Herstellung der Endprodukte

Beispiel 1

cis-2-(1,2,4-Triazol-1-yl-ethyl)-2-(3-trifluormethylphenyl)-3(4-fluorphenyl)-oxiran

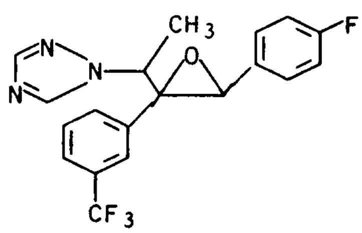

Eine Lösung von 7,73 g (0,11 mol) 1,2,4-Triazol in 100 ml N,N-Dimethylformamid wird mit 15,6 g Kaliumcarbonat versetzt und für 30 Minuten auf 50 °C erwärmt. Nachdem das Reaktionsgemisch auf Raumtemperatur gekühlt wurde, wird der Lösung 22,8 g (0,056 mol) cis-2-(1-Methylsulfonyloxyethyl)-2-(3-trifluormethylphenyl)-3-(4-fluorphenyl)-oxiran, das in 50 ml N,N-Dimethylformamid gelöst ist, langsam zugetropft und 13 Stunden bei Raumtemperatur gerührt. Anschließend wird 100 ml Wasser zugegeben und mehrmals mit Methyl-tert. -butylether extrahiert; die organische Phase mit Wasser gewaschen, über Natriumsulfat getrocknet und im Vakuum eingeengt.

Ausbeute: 7,7 g (36 %) (Verbindung Nr. 1)

Entsprechend Beispiel 1 können die in der Tabelle aufgeführten Verbindungen hergestellt werden.

Tabelle

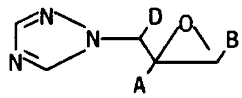

| Beispiel | A | B | D | X | Schmp./$^1$H-NMR [ppm] |
|---|---|---|---|---|---|
| 1 | $3-CF_3-C_6H_4$ | $4-F-C_6H_4$ | $CH_3$ | N | 1.68 (d), 3.72 (s), 5.04 (g), 8.04, 8.14 (2S) |
| 2 | $3-CF_3-C_6H_4$ | $4-F-C_6H_4$ | $CH_3$ | CH | 1.62 (d), 4.39 (s), 4.48 (g) |
| 3 | $3-CF_3-C_6H_4$ | $4-F-C_6H_4$ | $C_2H_5$ | N | |
| 4 | $3-CF_3-C_6H_4$ | $4-F-C_6H_4$ | $C_3H_7$ | N | |
| 5 | $3-CF_3-C_6H_4$ | $4-F-C_6H_4$ | $C_4H_9$ | N | |
| 6 | $3-CF_3-C_6H_4$ | $4-F-C_6H_4$ | $C_5H_{11}$ | N | |
| 7 | $3-CF_3-C_6H_4$ | $4-F-C_6H_4$ | $C_6H_{13}$ | N | |
| 8 | $3-CF_3-C_6H_4$ | $C_6H_5$ | $CH_3$ | N | |
| 9 | $3-CF_3-C_6H_4$ | $2-F-C_6H_4$ | $CH_3$ | N | |
| 10 | $3-CF_3-C_6H_4$ | $2-F-C_6H_4$ | $C_2H_5$ | N | |
| 11 | $3-CF_3-C_6H_4$ | $3-F-C_6H_4$ | $CH_3$ | N | |
| 12 | $3-CF_3-C_6H_4$ | $2-Cl-C_6H_4$ | $CH_3$ | N | |
| 13 | $3-CF_3-C_6H_4$ | $2-Cl-C_6H_4$ | $C_2H_5$ | N | |
| 14 | $3-CF_3-C_6H_4$ | $2-Cl-C_6H_4$ | $C_3H_7$ | N | |
| 15 | $3-CF_3-C_6H_4$ | $2-Cl-C_6H_4$ | $iso-C_3H_7$ | N | |
| 16 | $3-CF_3-C_6H_4$ | $3-Cl-C_6H_4$ | $CH_3$ | N | |
| 17 | $3-CF_3-C_6H_4$ | $4-Cl-C_6H_4$ | $CH_3$ | N | |
| 18 | $3-CF_3-C_6H_4$ | $4-Cl-C_6H_4$ | $C_2H_5$ | N | |
| 19 | $3-CF_3-C_6H_4$ | $4-Cl-C_6H_4$ | $C_3H_7$ | N | |
| 20 | $3-CF_3-C_6H_4$ | $2,4-Cl_2-C_6H_3$ | $CH_3$ | N | |
| 21 | $3-CF_3-C_6H_4$ | $2,4-Cl_2-C_6H_3$ | $C_2H_5$ | N | |
| 22 | $3-CF_3-C_6H_4$ | $2-CH_3-C_6H_4$ | $CH_3$ | N | |
| 23 | $3-CF_3-C_6H_4$ | $4-CH_3-C_6H_4$ | $CH_3$ | N | |
| 24 | $3-CF_3-C_6H_4$ | $2,4-(CH_3)_2-C_6H_3$ | $CH_3$ | N | |
| 25 | $3-CF_3-C_6H_4$ | $2-OCH_3-C_6H_4$ | $CH_3$ | N | |
| 26 | $3-CF_3-C_6H_4$ | $4-OCH_3-C_6H_4$ | $CH_3$ | N | |
| 27 | $3-CF_3-C_6H_4$ | $2-CF_3-C_6H_4$ | $CH_3$ | N | |

| Beispiel | A | B | D | X | Schmp./$^1$H-NMR [ppm] |
|---|---|---|---|---|---|
| 28 | 3-$CF_3$-$C_6H_4$ | 3-$CF_3$-$C_6H_4$ | $CH_3$ | N | |
| 29 | 3-$CF_3$-$C_6H_4$ | 4-$CF_3$-$C_6H_4$ | $CH_3$ | N | |
| 30 | 3-$CF_3$-$C_6H_4$ | 1-Naphthyl | $CH_3$ | N | |
| 31 | 3-$CF_3$-$C_6H_4$ | 2-Naphthyl | $CH_3$ | N | |
| 32 | 3-$CF_3$-$C_6H_4$ | 4-Biphenyl | $CH_3$ | N | |
| 33 | $C_6H_5$ | $C_6H_5$ | $CH_3$ | N | |
| 34 | $C_6H_5$ | 2-Cl-$C_6H_4$ | $CH_3$ | N | |
| 35 | $C_6H_5$ | 2-Cl-$C_6H_4$ | $C_2H_5$ | N | 0.90, 1.06 (2 t), 3.92 (s), 4.76 (dd), 5.14 (t), 7.99, 8.14 (2 s) |
| 36 | $C_6H_5$ | 2-Cl-$C_6H_4$ | $C_3H_7$ | N | |
| 37 | $C_6H_5$ | 3-Cl-$C_6H_4$ | $CH_3$ | N | |
| 38 | $C_6H_5$ | 4-Cl-$C_6H_4$ | $CH_3$ | N | |
| 39 | $C_6H_5$ | 4-Cl-$C_6H_4$ | $C_2H_5$ | N | |
| 40 | $C_6H_5$ | 4-Cl-$C_6H_4$ | $C_3H_7$ | N | |
| 41 | $C_6H_5$ | 2,4-$Cl_2$-$C_6H_3$ | $CH_3$ | N | |
| 42 | $C_6H_5$ | 2,4-$Cl_2$-$C_6H_3$ | $C_2H_5$ | N | |
| 43 | $C_6H_5$ | 2-F-$C_6H_4$ | $CH_3$ | N | |
| 44 | $C_6H_5$ | 2-F-$C_6H_4$ | $C_2H_5$ | N | |
| 45 | $C_6H_5$ | 3-F-$C_6H_4$ | $CH_3$ | N | |
| 46 | $C_6H_5$ | 4-F-$C_6H_4$ | $CH_3$ | N | |
| 47 | $C_6H_5$ | 4-F-$C_6H_4$ | $C_2H_5$ | N | |
| 48 | $C_6H_5$ | 4-F-$C_6H_4$ | $C_3H_7$ | N | |
| 49 | $C_6H_5$ | 2-Br-$C_6H_4$ | $CH_3$ | N | |
| 50 | $C_6H_5$ | 2-$CH_3$-$C_6H_4$ | $CH_3$ | N | |
| 51 | $C_6H_5$ | 4-$CH_3$-$C_6H_4$ | $CH_3$ | N | |
| 52 | $C_6H_5$ | 2-$OCH_3$-$C_6H_4$ | $CH_3$ | N | |
| 53 | $C_6H_5$ | 2-$CF_3$-$C_6H_4$ | $CH_3$ | N | |
| 54 | $C_6H_5$ | 3-$CF_3$-$C_6H_4$ | $CH_3$ | N | |
| 55 | $C_6H_5$ | 4-$CF_3$-$C_6H_4$ | $CH_3$ | N | |
| 56 | $C_6H_5$ | 3-$NO_2$-$C_6H_4$ | $CH_3$ | N | |
| 57 | $C_6H_5$ | 3-$NH_2$-$C_6H_4$ | $CH_3$ | N | |
| 58 | $C_6H_5$ | 1-Naphthyl | $CH_3$ | N | |
| 59 | $C_6H_5$ | 2-Naphthyl | $CH_3$ | N | |
| 60 | $C_6H_5$ | 4-Biphenyl | $CH_3$ | N | |
| 61 | $C_6H_5$ | $CH_3$ | $CH_3$ | N | |
| 62 | $C_6H_5$ | $C_2H_5$ | $CH_3$ | N | |

| Beispiel | A | B | D | X | Schmp./$^1$H-NMR [ppm] |
|---|---|---|---|---|---|
| 63 | $C_6H_5$ | Cyclopentyl | $CH_3$ | N | |
| 64 | $C_6H_5$ | Cyclohexyl | $CH_3$ | N | |
| 65 | $C_6H_5$ | 3-Cyclohexenyl | $CH_3$ | N | |
| 66 | $C_6H_5$ | 2-Furyl | $CH_3$ | N | |
| 67 | $C_6H_5$ | 2-Thienyl | $CH_3$ | N | |
| 68 | $C_6H_5$ | 3-Thienyl | $CH_3$ | N | |
| 69 | $C_6H_5$ | 2-Pyridyl | $CH_3$ | N | |
| 70 | $C_6H_5$ | 3-Pyridyl | $CH_3$ | N | |
| 71 | 2-Cl-$C_6H_4$ | $C_6H_5$ | $CH_3$ | N | |
| 72 | 2-Cl-$C_6H_4$ | 2-Cl-$C_6H_4$ | $CH_3$ | N | |
| 73 | 2-Cl-$C_6H_4$ | 3-Cl-$C_6H_4$ | $CH_3$ | N | |
| 74 | 2-Cl-$C_6H_4$ | 4-Cl-$C_6H_4$ | $CH_3$ | N | |
| 75 | 2-Cl-$C_6H_4$ | 2,4-$Cl_2$-$C_6H_3$ | $CH_3$ | N | |
| 76 | 2-Cl-$C_6H_4$ | 2-F-$C_6H_4$ | $CH_3$ | N | |
| 77 | 2-Cl-$C_6H_4$ | 4-F-$C_6H_4$ | $CH_3$ | N | |
| 78 | 2-Cl-$C_6H_4$ | 2-Br-$C_6H_4$ | $CH_3$ | N | |
| 79 | 2-Cl-$C_6H_4$ | 2-$CH_3$-$C_6H_4$ | $CH_3$ | N | |
| 80 | 2-Cl-$C_6H_4$ | 4-$CH_3$-$C_6H_4$ | $CH_3$ | N | |
| 81 | 2-Cl-$C_6H_4$ | 2-$OCH_3$-$C_6H_4$ | $CH_3$ | N | |
| 82 | 2-Cl-$C_6H_4$ | 2-$CF_3$-$C_6H_4$ | $CH_3$ | N | |
| 83 | 2-Cl-$C_6H_4$ | 4-$CF_3$-$C_6H_4$ | $CH_3$ | N | |
| 84 | 2-Cl-$C_6H_4$ | 2-Furyl | $CH_3$ | N | |
| 85 | 2-Cl-$C_6H_4$ | 2-Thienyl | $CH_3$ | N | |
| 86 | 2-Cl-$C_6H_4$ | 3-Pyridyl | $CH_3$ | N | |
| 87 | 3-Cl-$C_6H_4$ | $C_6H_5$ | $CH_3$ | N | |
| 88 | 3-Cl-$C_6H_4$ | 2-Cl-$C_6H_4$ | $CH_3$ | N | |
| 89 | 3-Cl-$C_6H_4$ | 4-Cl-$C_6H_4$ | $CH_3$ | N | |
| 90 | 3-Cl-$C_6H_4$ | 2,4-$Cl_2$-$C_6H_3$ | $CH_3$ | N | |
| 91 | 3-Cl-$C_6H_4$ | 2-F-$C_6H_4$ | $CH_3$ | N | |
| 92 | 3-Cl-$C_6H_4$ | 4-F-$C_6H_4$ | $CH_3$ | N | |
| 93 | 3-Cl-$C_6H_4$ | 2-$CF_3$-$C_6H_4$ | $CH_3$ | N | |
| 94 | 3-Cl-$C_6H_4$ | 4-$CF_3$-$C_6H_4$ | $CH_3$ | N | |
| 95 | 4-Cl-$C_6H_4$ | $C_6H_5$ | $CH_3$ | N | |
| 96 | 4-Cl-$C_6H_4$ | 2-Cl-$C_6H_4$ | $CH_3$ | N | |
| 97 | 4-Cl-$C_6H_4$ | 2-Cl-$C_6H_4$ | $C_2H_5$ | N | |

| Beispiel | A | B | D | X | Schmp./$^1$H-NMR [ppm] |
|---|---|---|---|---|---|
| 98 | 4-Cl-$C_6H_4$ | 2-Cl-$C_6H_4$ | $C_3H_7$ | N | |
| 99 | 4-Cl-$C_6H_4$ | 2-Cl-$C_6H_4$ | iso-$C_3H_7$ | N | |
| 100 | 4-Cl-$C_6H_4$ | 2-Cl-$C_6H_4$ | $C_4H_9$ | N | |
| 101 | 4-Cl-$C_6H_4$ | 3-Cl-$C_6H_4$ | $CH_3$ | N | |
| 102 | 4-Cl-$C_6H_4$ | 4-Cl-$C_6H_4$ | $CH_3$ | N | |
| 103 | 4-Cl-$C_6H_4$ | 4-Cl-$C_6H_4$ | $C_2H_5$ | N | |
| 104 | 4-Cl-$C_6H_4$ | 4-Cl-$C_6H_4$ | $C_3H_7$ | N | |
| 105 | 4-Cl-$C_6H_4$ | 2,4-$Cl_2$-$C_6H_3$ | $CH_3$ | N | |
| 106 | 4-Cl-$C_6H_4$ | 2,4-$Cl_2$-$C_6H_3$ | $C_2H_5$ | N | |
| 107 | 4-Cl-$C_6H_4$ | 2-F-$C_6H_4$ | $CH_3$ | N | |
| 108 | 4-Cl-$C_6H_4$ | 4-F-$C_6H_4$ | $CH_3$ | N | |
| 109 | 4-Cl-$C_6H_4$ | 2-Br-$C_6H_4$ | $CH_3$ | N | |
| 110 | 4-Cl-$C_6H_4$ | 2-$CH_3$-$C_6H_4$ | $CH_3$ | N | |
| 111 | 4-Cl-$C_6H_4$ | 4-$CH_3$-$C_6H_4$ | $CH_3$ | N | |
| 112 | 4-Cl-$C_6H_4$ | 2-$OCH_3$-$C_6H_4$ | $CH_3$ | N | |
| 113 | 4-Cl-$C_6H_4$ | 2-$CF_3$-$C_6H_4$ | $CH_3$ | N | |
| 114 | 4-Cl-$C_6H_4$ | 3-$CF_3$-$C_6H_4$ | $CH_3$ | N | |
| 115 | 4-Cl-$C_6H_4$ | 4-$CF_3$-$C_6H_4$ | $CH_3$ | N | |
| 116 | 4-Cl-$C_6H_4$ | 3-$NO_2$-$C_6H_4$ | $CH_3$ | N | |
| 117 | 4-Cl-$C_6H_4$ | 1-Naphthyl | $CH_3$ | N | |
| 118 | 4-Cl-$C_6H_4$ | 2-Naphthyl | $CH_3$ | N | |
| 119 | 4-Cl-$C_6H_4$ | 4-Biphenyl | $CH_3$ | N | |
| 120 | 4-Cl-$C_6H_4$ | $CH_3$ | $CH_3$ | N | |
| 121 | 4-Cl-$C_6H_4$ | $C_2H_5$ | $CH_3$ | N | |
| 122 | 4-Cl-$C_6H_4$ | Cyclopentyl | $CH_3$ | N | |
| 123 | 4-Cl-$C_6H_4$ | Cyclohexyl | $CH_3$ | N | |
| 124 | 4-Cl-$C_6H_4$ | 2-Furyl | $CH_3$ | N | |
| 125 | 4-Cl-$C_6H_4$ | 2-Thienyl | $CH_3$ | N | |
| 126 | 4-Cl-$C_6H_4$ | 3-Thienyl | $CH_3$ | N | |
| 127 | 4-Cl-$C_6H_4$ | 2-Pyridyl | $CH_3$ | N | |
| 128 | 4-Cl-$C_6H_4$ | 3-Pyridyl | $CH_3$ | N | |
| 129 | 2-F-$C_6H_4$ | $C_6H_5$ | $CH_3$ | N | |
| 130 | 2-F-$C_6H_4$ | 2-Cl-$C_6H_4$ | $CH_3$ | N | |
| 131 | 2-F-$C_6H_4$ | 4-Cl-$C_6H_4$ | $CH_3$ | N | |

| Beispiel | A | B | D | X | Schmp./$^1$H-NMR [ppm] |
|---|---|---|---|---|---|
| 132 | 2-F-$C_6H_4$ | 2,4-$Cl_2$-$C_6H_3$ | $CH_3$ | N | |
| 133 | 2-F-$C_6H_4$ | 2-F-$C_6H_4$ | $CH_3$ | N | |
| 134 | 2-F-$C_6H_4$ | 4-F-$C_6H_4$ | $CH_3$ | N | |
| 135 | 2-F-$C_6H_4$ | 2-$CF_3$-$C_6H_4$ | $CH_3$ | N | |
| 136 | 2-F-$C_6H_4$ | 4-$CF_3$-$C_6H_4$ | $CH_3$ | N | |
| 137 | 3-F-$C_6H_4$ | 2-Cl-$C_6H_4$ | $CH_3$ | N | |
| 138 | 3-F-$C_6H_4$ | 4-Cl-$C_6H_4$ | $CH_3$ | N | |
| 139 | 3-F-$C_6H_4$ | 4-F-$C_6H_4$ | $CH_3$ | N | |
| 140 | 4-F-$C_6H_4$ | $C_6H_5$ | $CH_3$ | N | |
| 141 | 4-F-$C_6H_4$ | 2-Cl-$C_6H_4$ | $CH_3$ | N | |
| 142 | 4-F-$C_6H_4$ | 2-Cl-$C_6H_4$ | $C_2H_5$ | N | |
| 143 | 4-F-$C_6H_4$ | 2-Cl-$C_6H_4$ | $C_3H_7$ | N | |
| 144 | 4-F-$C_6H_4$ | 2-Cl-$C_6H_4$ | iso-$C_3H_7$ | N | |
| 145 | 4-F-$C_6H_4$ | 2-Cl-$C_6H_4$ | $C_4H_9$ | N | |
| 146 | 4-F-$C_6H_4$ | 2-Cl-$C_6H_4$ | $C_5H_{11}$ | N | |
| 147 | 4-F-$C_6H_4$ | 3-Cl-$C_6H_4$ | $CH_3$ | N | |
| 148 | 4-F-$C_6H_4$ | 4-Cl-$C_6H_4$ | $CH_3$ | N | |
| 149 | 4-F-$C_6H_4$ | 4-Cl-$C_6H_4$ | $C_2H_5$ | N | |
| 150 | 4-F-$C_6H_4$ | 4-Cl-$C_6H_4$ | $C_3H_7$ | N | |
| 151 | 4-F-$C_6H_4$ | 4-Cl-$C_6H_4$ | $C_4H_9$ | N | |
| 152 | 4-F-$C_6H_4$ | 2,4-$Cl_2$-$C_6H_3$ | $CH_3$ | N | |
| 153 | 4-F-$C_6H_4$ | 2,4-$Cl_2$-$C_6H_3$ | $CH_3$ | N | |
| 154 | 4-F-$C_6H_4$ | 2-F-$C_6H_4$ | $CH_3$ | N | |
| 155 | 4-F-$C_6H_4$ | 4-F-$C_6H_4$ | $CH_3$ | N | |
| 156 | 4-F-$C_6H_4$ | 2-Br-$C_6H_4$ | $CH_3$ | N | |
| 157 | 4-F-$C_6H_4$ | 2-$CH_3$-$C_6H_4$ | $CH_3$ | N | |
| 158 | 4-F-$C_6H_4$ | 4-$CH_3$-$C_6H_4$ | $CH_3$ | N | |
| 159 | 4-F-$C_6H_4$ | 2,4-$(CH_3)_2$-$C_6H_3$ | $CH_3$ | N | |
| 160 | 4-F-$C_6H_4$ | 2-$OCH_3$-$C_6H_4$ | $CH_3$ | N | |
| 161 | 4-F-$C_6H_4$ | 4-$OCH_3$-$C_6H_4$ | $CH_3$ | N | |
| 162 | 4-F-$C_6H_4$ | 2-$CF_3$-$C_6H_4$ | $CH_3$ | N | |
| 163 | 4-F-$C_6H_4$ | 4-$CF_3$-$C_6H_4$ | $CH_3$ | N | |
| 164 | 4-F-$C_6H_4$ | 3-$NO_2$-$C_6H_4$ | $CH_3$ | N | |
| 165 | 4-F-$C_6H_4$ | 3-$NH_2$-$C_6H_4$ | $CH_3$ | N | |
| 166 | 4-F-$C_6H_4$ | 1-Naphthyl | $CH_3$ | N | |

| Beispiel | A | B | D | X | Schmp./$^1$H-NMR [ppm] |
|---|---|---|---|---|---|
| 167 | $4-F-C_6H_4$ | 2-Naphthyl | $CH_3$ | N | |
| 168 | $4-F-C_6H_4$ | 4-Biphenyl | $CH_3$ | N | |
| 169 | $4-F-C_6H_4$ | $CH_3$ | $CH_3$ | N | |
| 170 | $4-F-C_6H_4$ | $C_2H_5$ | $CH_3$ | N | |
| 171 | $4-F-C_6H_4$ | Cyclopentyl | $CH_3$ | N | |
| 172 | $4-F-C_6H_4$ | Cyclohexyl | $CH_3$ | N | |
| 173 | $4-F-C_6H_4$ | 2-Furyl | $CH_3$ | N | |
| 174 | $4-F-C_6H_4$ | 2-Thienyl | $CH_3$ | N | |
| 175 | $4-F-C_6H_4$ | 3-Thienyl | $CH_3$ | N | |
| 176 | $4-F-C_6H_4$ | 2-Pyridyl | $CH_3$ | N | |
| 177 | $4-F-C_6H_4$ | 3-Pyridyl | $CH_3$ | N | |
| 178 | $4-F-C_6H_4$ | 4-Pyridyl | $CH_3$ | N | |
| 179 | $2,4-Cl_2-C_6H_3$ | $C_6H_5$ | $CH_3$ | N | |
| 180 | $2,4-Cl_2-C_6H_3$ | $2-Cl-C_6H_4$ | $CH_3$ | N | |
| 181 | $2,4-Cl_2-C_6H_3$ | $3-Cl-C_6H_4$ | $CH_3$ | N | |
| 182 | $2,4-Cl_2-C_6H_3$ | $4-Cl-C_6H_4$ | $CH_3$ | N | |
| 183 | $2,4-Cl_2-C_6H_3$ | $2,4-Cl_2-C_6H_4$ | $CH_3$ | N | |
| 184 | $2,4-Cl_2-C_6H_3$ | $2-F-C_6H_4$ | $CH_3$ | N | |
| 185 | $2,4-Cl_2-C_6H_3$ | $4-F-C_6H_4$ | $CH_3$ | N | |
| 186 | $2,4-Cl_2-C_6H_3$ | $2-CF_3-C_6H_4$ | $CH_3$ | N | |
| 187 | $2,4-Cl_2-C_6H_3$ | $4-CF_3-C_6H_4$ | $CH_3$ | N | |
| 188 | $2,4-Cl_2-C_6H_3$ | $2-CH_3-C_6H_4$ | $CH_3$ | N | |
| 189 | $2,4-Cl_2-C_6H_3$ | $4-CH_3-C_6H_4$ | $CH_3$ | N | |
| 190 | $4-Br-C_6H_4$ | $C_6H_5$ | $CH_3$ | N | |
| 191 | $4-Br-C_6H_4$ | $2-Cl-C_6H_4$ | $CH_3$ | N | |
| 192 | $4-Br-C_6H_4$ | $4-Cl-C_6H_4$ | $CH_3$ | N | |
| 193 | $4-Br-C_6H_4$ | $2,4-Cl_2-C_6H_3$ | $CH_3$ | N | |
| 194 | $4-Br-C_6H_4$ | $4-F-C_6H_4$ | $CH_3$ | N | |
| 195 | $4-Br-C_6H_4$ | $2-CF_3-C_6H_4$ | $CH_3$ | N | |
| 196 | $2-CH_3-C_6H_4$ | $C_6H_5$ | $CH_3$ | N | |
| 197 | $2-CH_3-C_6H_4$ | $2-Cl-C_6H_4$ | $CH_3$ | N | |
| 198 | $2-CH_3-C_6H_4$ | $4-Cl-C_6H_4$ | $CH_3$ | N | |
| 199 | $2-CH_3-C_6H_4$ | $4-F-C_6H_4$ | $CH_3$ | N | |
| 200 | $2-CH_3-C_6H_4$ | $2-CF_3-C_6H_4$ | $CH_3$ | N | |
| 201 | $4-CH_3-C_6H_4$ | $C_6H_5$ | $CH_3$ | N | |

| Beispiel | A | B | D | X | Schmp./$^1$H-NMR [ppm] |
|---|---|---|---|---|---|
| 202 | $4-CH_3-C_6H_4$ | $2-Cl-C_6H_4$ | $CH_3$ | N | |
| 203 | $4-CH_3-C_6H_4$ | $4-Cl-C_6H_4$ | $CH_3$ | N | |
| 204 | $4-CH_3-C_6H_4$ | $2,4-Cl_2-C_6H_3$ | $CH_3$ | N | |
| 205 | $4-CH_3-C_6H_4$ | $2-F-C_6H_4$ | $CH_3$ | N | |
| 206 | $4-CH_3-C_6H_4$ | $4-F-C_6H_4$ | $CH_3$ | N | |
| 207 | $4-CH_3-C_6H_4$ | $2-CF_3-C_6H_4$ | $CH_3$ | N | |
| 208 | $4-CH_3-C_6H_4$ | $4-CF_3-C_6H_4$ | $CH_3$ | N | |
| 209 | $4-CH_3-C_6H_4$ | $2-CH_3-C_6H_4$ | $CH_3$ | N | |
| 210 | $2-OCH_3-C_6H_4$ | $C_6H_5$ | $CH_3$ | N | |
| 211 | $2-OCH_3-C_6H_4$ | $2-Cl-C_6H_4$ | $CH_3$ | N | |
| 212 | $2-OCH_3-C_6H_4$ | $2-F-C_6H_4$ | $CH_3$ | N | |
| 213 | $2-OCH_3-C_6H_4$ | $4-F-C_6H_4$ | $CH_3$ | N | |
| 214 | $2-OCH_3-C_6H_4$ | $4-Cl-C_6H_4$ | $CH_3$ | N | |
| 215 | $4-OCH_3-C_6H_4$ | $C_6H_5$ | $CH_3$ | N | |
| 216 | $4-OCH_3-C_6H_4$ | $2-Cl-C_6H_4$ | $CH_3$ | N | 1.72, 1.79 (2 d), 3.66, 3.82 (2 s), 3.94, 4.58 (2 s), 7.94, 7.98, 8.04, 8.12 (4s) |
| 217 | $4-OCH_3-C_6H_4$ | $4-Cl-C_6H_4$ | $CH_3$ | N | |
| 218 | $4-OCH_3-C_6H_4$ | $2-F-C_6H_4$ | $CH_3$ | N | |
| 219 | $4-OCH_3-C_6H_4$ | $4-F-C_6H_4$ | $CH_3$ | N | |
| 220 | $2-CF_3-C_6H_4$ | $C_6H_5$ | $CH_3$ | N | |
| 221 | $2-CF_3-C_6H_4$ | $2-Cl-C_6H_4$ | $CH_3$ | N | |
| 222 | $2-CF_3-C_6H_4$ | $4-Cl-C_6H_4$ | $CH_3$ | N | |
| 223 | $2-CF_3-C_6H_4$ | $2-F-C_6H_4$ | $CH_3$ | N | |
| 224 | $2-CF_3-C_6H_4$ | $4-F-C_6H_4$ | $CH_3$ | N | |
| 225 | $4-CF_3-C_6H_4$ | $C_6H_5$ | $CH_3$ | N | |
| 226 | $4-CF_3-C_6H_4$ | $2-Cl-C_6H_4$ | $CH_3$ | N | |
| 227 | $4-CF_3-C_6H_4$ | $4-Cl-C_6H_4$ | $CH_3$ | N | |
| 228 | $4-CF_3-C_6H_4$ | $2-F-C_6H_4$ | $CH_3$ | N | |
| 229 | $4-CF_3-C_6H_4$ | $4-F-C_6H_4$ | $CH_3$ | N | |
| 230 | 1-Naphthyl | $2-Cl-C_6H_4$ | $CH_3$ | N | |
| 231 | 1-Naphthyl | $4-Cl-C_6H_4$ | $CH_3$ | N | |
| 232 | 1-Naphthyl | $4-F-C_6H_4$ | $CH_3$ | N | |
| 233 | 2-Naphthyl | $2-Cl-C_6H_4$ | $CH_3$ | N | |
| 234 | 2-Naphthyl | $4-Cl-C_6H_4$ | $CH_3$ | N | |
| 235 | 2-Naphthyl | $4-F-C_6H_4$ | $CH_3$ | N | |
| 236 | 4-Biphenyl | $2-Cl-C_6H_4$ | $CH_3$ | N | |

| Beispiel | A | B | D | X | Schmp./$^1$H-NMR [ppm] |
|---|---|---|---|---|---|
| 237 | 4-Biphenyl | 4-Cl-$C_6H_4$ | $CH_3$ | N | |
| 238 | $CH_3$ | $C_6H_5$ | $CH_3$ | N | |
| 239 | $CH_3$ | 2-Cl-$C_6H_4$ | $CH_3$ | N | |
| 240 | $CH_3$ | 4-Cl-$C_6H_4$ | $CH_3$ | N | |
| 241 | $CH_3$ | 2-F-$C_6H_4$ | $CH_3$ | N | |
| 242 | $CH_3$ | 4-F-$C_6H_4$ | $CH_3$ | N | |
| 243 | $CH_3$ | 2,4-$Cl_2$-$C_6H_3$ | $CH_3$ | N | |
| 244 | Cyclohexyl | $C_6H_5$ | $CH_3$ | N | |
| 245 | Cyclohexyl | 2-Cl-$C_6H_4$ | $CH_3$ | N | |
| 246 | Cyclohexyl | 4-Cl-$C_6H_4$ | $CH_3$ | N | |
| 247 | Cyclohexyl | 2,4-$Cl_2$-$C_6H_3$ | $CH_3$ | N | |
| 248 | Cyclohexyl | 2-F-$C_6H_4$ | $CH_3$ | N | |
| 249 | Cyclohexyl | 4-F-$C_6H_4$ | $CH_3$ | N | |
| 250 | 2-Furyl | $C_6H_5$ | $CH_3$ | N | |
| 251 | 2-Furyl | 2-Cl-$C_6H_4$ | $CH_3$ | N | |
| 252 | 2-Furyl | 2,4-$Cl_2$-$C_6H_3$ | $CH_3$ | N | |
| 253 | 2-Furyl | 4-Cl-$C_6H_4$ | $CH_3$ | N | |
| 254 | 2-Furyl | 2-F-$C_6H_4$ | $CH_3$ | N | |
| 255 | 2-Furyl | 4-F-$C_6H_4$ | $CH_3$ | N | |
| 256 | 2-Thienyl | $C_6H_5$ | $CH_3$ | N | |
| 257 | 2-Thienyl | 2-Cl-$C_6H_4$ | $CH_3$ | N | |
| 258 | 2-Thienyl | 4-Cl-$C_6H_4$ | $CH_3$ | N | |
| 259 | 2-Thienyl | 2,4-$Cl_2$-$C_6H_3$ | $CH_3$ | N | |
| 260 | 2-Thienyl | 2-F-$C_6H_4$ | $CH_3$ | N | |
| 261 | 2-Thienyl | 4-F-$C_6H_4$ | $CH_3$ | N | |
| 262 | 3-Thienyl | $C_6H_5$ | $CH_3$ | N | |
| 263 | 3-Thienyl | 2-Cl-$C_6H_4$ | $CH_3$ | N | |
| 264 | 3-Thienyl | 4-Cl-$C_6H_4$ | $CH_3$ | N | |
| 265 | 3-Thienyl | 2,4-$Cl_2$-$C_6H_3$ | $CH_3$ | N | |
| 266 | 3-Thienyl | 4-F-$C_6H_4$ | $CH_3$ | N | |
| 267 | 4-Pyridyl | $C_6H_5$ | $CH_3$ | N | |
| 268 | 4-Pyridyl | 2-Cl-$C_6H_4$ | $CH_3$ | N | |
| 269 | 4-Pyridyl | 4-Cl-$C_6H_4$ | $CH_3$ | N | |
| 270 | 4-Pyridyl | 2,4-$Cl_2$-$C_6H_3$ | $CH_3$ | N | |
| 271 | 4-Pyridyl | 4-F-$C_6H_4$ | $CH_3$ | N | |

Die neuen Verbindungen eignen sich als Fungizide.

Die neuen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet

werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Nr. 1 und 10 Gew.-Teilen N-Methyl-$\alpha$-pyrrolidon, die zur Anwendung in Form kleinster Tropfen geeignet ist;

II. eine Mischung aus 20 Gew.-Teilen der Verbindung Nr. 1, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 1, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 1, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 1, 3 Gew.-Teilen des Natriumsalzes der Diisobutylnaphtalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 1 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 1, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 1, 10 Gew.-Teilen des Natriumsalzes eines Phenosulfonsäureharnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 1, 2 Gew.-Teilen des Calciumsalzes der Dodecylbenzolsulfonsäure, 8 Gew.-Teilen Fettalkohol-polyglykolether, 20 Gew.-Teilen des

Natriumsalzes eines Phenolsulfonsäure-harnstoff-formaldehyd-Kondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

## Patentansprüche

**1.** Azolylmethyloxirane der allgemeinen Formel I

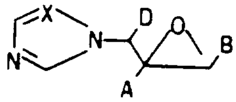

I

in welcher

A und B $C_1$-$C_8$-Alkyl, $C_3$-$C_8$-Cycloalkyl, $C_5$-$C_8$-Cycloalkenyl, Biphenyl, Naphthyl, 5-gliedriges Heteroaryl mit einem oder Zwei der Heteroatome O, S und N, 6-gliedriges Heteroaryl mit bis zu drei Stickstoffatome oder Phenyl bedeuten, wobei jeder dieser Reste ein- bis dreifach durch Halogen, Nitro, Phenoxy, Amino, $C_1$-$C_4$-Alkyl, $C_1$-$C_4$-Alkoxy oder $C_1$-$C_4$-Halogenalkyl substituiert sein kann,

D den Rest $C_1$-$C_8$-Alkyl bedeutet,

X den Rest CH oder N bedeutet,

sowie deren für Pflanzen verträgliche Säureadditionssalze und Metallkomplexe.

**2.** Azolylmethyloxirane der allgemeinen Formel I nach Anspruch 1, in denen A und B die Phenylgruppe bedeuten, welche unsubstituiert, oder durch einen oder zwei Substituenten, ausgewählt aus der Gruppe Fluor, Chlor, Brom und Trifluormethyl, substituiert ist.

**3.** Verfahren zur Herstellung der Azolylmethyloxirane der allgemeinen Formel I gemäß Anspruch 1 sowie gegebenenfalls ihrer für Pflanzen verträglichen Säureadditionssalze oder Metallkomplexe, dadurch gekennzeichnet, daß man

a) eine Verbindung der allgemeinen Formel II

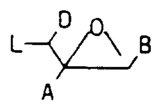

II

in welcher L eine nukleophile Abgangsgruppe darstellt, mit einer Verbindung der allgemeinen Formel III

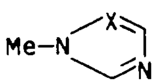

III

in der Me ein Wasserstoffatom oder ein Metallatom bedeutet, umsetzt oder

b) eine Verbindung der allgemeinen Formel IV

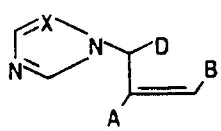

IV

durch Epoxidierung in das entsprechende Oxiran überführt und die so erhaltenen Verbindungen gegebenenfalls in ihre Salze mit für Pflanzen verträglichen Säuren oder Metallkomplexe überführt.

**4.** Fungizides Mittel, enthaltend einen Trägerstoff und eine fungizid wirksame Menge eines Azolylmethyloxirans der allgemeinen Formel I gemäß Anspruch 1 oder dessen für Pflanzen verträgliches Säureadditionssalz und Metallkomplexes.

**5.** Verfahren zur Bekämpfung von Pilzen, dadurch gekennzeichnet, daß man eine fungizid wirksame Menge eines Azolylmethyloxirans der allgemeinen Formel I gemäß Anspruch 1 oder dessen für Pflanzen verträglichen Säureadditionssalzes oder Metallkomplexes auf die Pilze oder auf durch Pilzbefall bedrohte Materialien, Flächen, Pflanzen oder Saatgüter einwirken läßt.

**6.** Verbindungen der allgemeinen Formel II

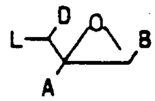

II

in welcher A, B und D die in Anspruch 1 angegebenen Bedeutungen haben und L eine nukleophile Abgangsgruppe darstellt, ausgenommen

a) 3,4-Epoxy-3-methyl-2-brompentan,
b) 3,4-Epoxy-3-methyl-2-(4-methylphenylsulfonyloxy)-pentan,
c) 3,4-Epoxy-3-methyl-2-pentanol,
d) 4,5-Epoxy-4-methyl-3-heptanol und
e) 3,4-Diphenyl-3,4-epoxy-2-butanol.

**7.** Verbindung der allgemeinen Formel I gemäß Anspruch 1, in der A 3-Trifluormethylphenyl, B 4-Fluorphenyl, D Methyl und X N bedeuten.

## Claims

**1.** An azolylmethyloxirane of the formula I

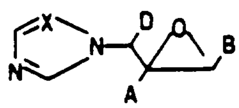

I

where A and B are each $C_1$-$C_8$-alkyl, $C_3$-$C_8$-cycloalkyl, $C_5$-$C_8$-cycloalkenyl, biphenyl, naphthyl, 5-membered hetaryl having one or two of the hetero atoms O, S, and N, 6-membered hetaryl having up to three nitrogen atoms, or phenyl, where each of these radicals may be monosubstituted to trisubstituted by halogen, nitro, phenoxy, amino, $C_1$-$C_4$-alkyl, $C_1$-$C_4$-alkoxy or $C_1$-$C_4$-haloalkyl, D is $C_1$-$C_8$-alkyl and X is CH or N, and its plant-tolerated acid addition salts and metal complexes.

**2.** An azolylmethyloxirane of the formula I as claimed in claim 1, wherein A and B are each phenyl which is unsubstituted or substituted by one or two substituents selected from the group consisting of fluorine, chlorine, bromine and trifluoromethyl.

**3.** A process for the preparation of an azolylmethyloxirane of the formula I as claimed in claim 1, and its plant-tolerated acid addition salts or metal complexes, wherein

a) a compound of the formula II

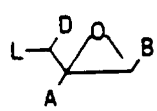

II

where L is a nucleophilic leaving group, is reacted with a compound of the formula III

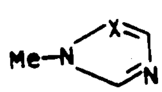

III

where Me is hydrogen or a metal atom, or

b) a compound of the formula IV

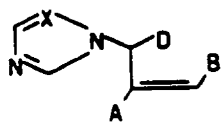

IV

is converted into the corresponding oxirane by epoxidation, and, if desired, the resulting compound is converted into its salts with plant-tolerated acids or metal complexes.

**4.** A fungicide containing a carrier and a fungicidal amount of an azolylmethyloxirane of the formula I as claimed in claim 1, or its plant-tolerated acid addition salt and metal complex.

**5.** A method for controlling fungi, wherein a fungicidal amount of an azolylmethyloxirane of the formula I as claimed in claim 1, or of its plant-tolerated acid addition salt or metal complex, is allowed to act on the fungi or on the materials, surfaces, plants or seeds threatened by fungal attack.

**6.** A compound of the formula II

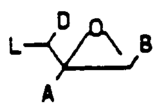

II

where A, B and D have the meanings stated in claim 1 and L is a nucleophilic leaving group, excluding
a) 3,4-epoxy-3-methyl-2-bromopentane,
b) 3,4-epoxy-3-methyl-2-(4-methylphenylsulfonyloxy)pentane,
c) 3,4-epoxy-3-methyl-2-pentanol,
d) 4,5-epoxy-4-methyl-3-heptanol and
e) 3,4-diphenyl-3,4-epoxy-2-butanol.

**7.** A compound of the formula I as claimed in claim 1, wherein A is 3-trifluoromethylphenyl, B is 4-fluoro1, wherein A is 3-trifluoromethylphenyl, B is 4-fluorophenyl, D is methyl and X is N.

## Revendications

**1.** Azolylméthyloxiranes de formule générale I

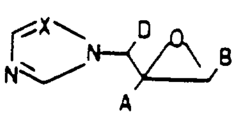

I

dans laquelle

| | |
|---|---|
| A et B | représentent alkyle en C1-C8, cycloalkyle en C3-C8, cycloalcényle en C5-C8, biphényle, naphtyle, hétéroaryle à 5 chaînons avec un ou deux hétéroatomes O, S et N, hétéroaryle à 6 chaînons avec jusqu'à trois atomes d'azote ou phényle, chacun de ces restes pouvant être substitué une à trois fois par halogène, nitro, phénoxy, amino, alkyle en C1-C4, alcoxy en C1-C4 ou halogénoalkyle en C1-C4, |
| D | représente le reste alkyle en C1-C8 |
| X | représente le reste CH ou N |

ainsi que leurs sels d'addition d'acide et complexes métalliques acceptables pour les plantes.

**2.** Azolylméthyloxiranes de formule générale I selon la revendication 1, dans laquelle A et B représentent le groupe phényle qui n'est pas substitué ou qui est substitué par un ou deux substituants choisis dans le groupe fluor, chlore, brome et trifluorométhyle.

**3.** Procédé de préparation d'azolylméthyloxiranes de formule générale I selon la revendication 1, ainsi qu'éventuellement leurs sels d'addition d'acide et complexes métalliques acceptables pour les plantes, caractérisé par le fait que

a) on fait réagir un composé de formule générale II

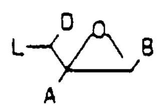

II

dans laquelle L représente un groupe éliminable nucléophile, avec un composé de formule générale III

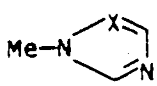

III

dans laquelle Me représente un atome d'hydrogène ou un atome de métal, ou bien

b) on transforme par époxydation un composé de formule générale IV

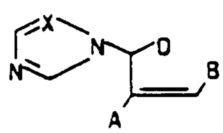

IV

en l'oxirane correspondant et on transforme les composés ainsi obtenus éventuellement en leurs sels d'addition d'acide et complexes métalliques acceptables pour les plantes.

**4.** Fongicide contenant un support et une quantité à action fongicide d'un azolylméthyloxirane de formule générale I selon la revendication 1 ou ses sels d'addition d'acide et complexes métalliques acceptables pour les plantes.

**5.** Procédé pour lutter contre les champignons, caractérisé par le fait que l'on fait agir une quantité à action fongicide d'un azolylméthyloxirane de formule générale I selon la revendication 1, ou ses sels d'addition d'acide et complexes métalliques acceptables pour les plantes, sur les champignons ou sur les matériaux, surfaces, plantes ou semences menacés par une attaque par les champignons.

**6.** Composé de formule générale II

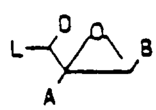

II

dans laquelle A, B et D ont les significations indiquées dans la revendication 1 et L représente un groupe éliminable nucléophile, à l'exception de

a) 3,4-époxy-3-méthyl-2-bromopentane,

b) 3,4-époxy-3-méthyl-2-(4-méthylphénylsulfonyloxy)-pentane,

c) 3,4-époxy-3-méthyl-2-pentanol,
d) 4,5-époxy-4-méthyl-3-heptanol et
e) 3,4-diphényl-3,4-époxy-2-butanol.

**7.** Composé de formule générale I selon la revendication 1, dans laquelle A représente 3-trifluorométhylphényle, B 4-fluorophényle, D méthyle et X N.